(19)

**Europäisches Patentamt**
**European Patent Office**
**Office européen des brevets**

(11)  **EP 2 235 132 B1**

(12)  **EUROPEAN PATENT SPECIFICATION**

(45) Date of publication and mention
of the grant of the patent:
**28.03.2018 Bulletin 2018/13**

(51) Int Cl.:
*C09J 7/04* *(0000.00)*      *C09J 11/00* *(2006.01)*
*A61L 15/58* *(2006.01)*

(21) Application number: **09700468.3**

(86) International application number:
**PCT/DK2009/050010**

(22) Date of filing: **08.01.2009**

(87) International publication number:
**WO 2009/086840 (16.07.2009 Gazette 2009/29)**

(54) **A PRESSURE SENSITIVE ADHESIVE COMPOSITION COMPRISING A WATER SOLUBLE COMPOUND**

DRUCKEMPFINDLICHE KLEBSTOFFZUSAMMENSETZUNG, DIE EINE WASSERLÖSLICHE VERBINDUNG ENTHÄLT

COMPOSITION ADHÉSIVE SENSIBLE À LA PRESSION COMPRENANT UN COMPOSÉ SOLUBLE DANS L'EAU

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **10.01.2008 DK 200800035**

(43) Date of publication of application:
**06.10.2010 Bulletin 2010/40**

(73) Proprietor: **Coloplast A/S**
**3050 Humlebæk (DK)**

(72) Inventors:
• **BACH, Anders**
**DK-2300 Koebenhavn S (DK)**
• **LYKKE, Mads**
**DK-2700 Broenshoej (DK)**

(56) References cited:
**EP-A- 0 628 320      EP-A- 1 148 106**
**WO-A-94/07468      WO-A2-2007/128320**

## Description

### Field of invention

The invention relates to a novel absorbing pressure sensitive adhesive composition comprising water soluble compound and medical devices comprising said absorbing adhesive composition.

### Background

Pressure sensitive adhesives have for a long time been used for attaching medical devices, such as ostomy appliances, dressings (including wound dressings), wound drainage bandages, devices for collecting urine, orthoses and prostheses to the skin.

It has been reported that humans for short periods can sweat more than 20,000 $g/m^2/24$ h, (Main, K., K. O. Nilsson, and N. E. Skakkebaek, 1991, Influence of sex and growth hormone deficiency on sweating, Scand. J. Clin. Lab Invest 51:475-480).

Thus, the moisture handling ability of skin contact adhesives, i.e. both the water absorption capacity and the moisture vapour transmission rate of the adhesive, is important.

When designing a skin adhesive one of the major issues is to keep the skin relatively dry underneath the adhesive to prevent maceration. Maceration occurs when skin is unable to get rid of moisture from transpiration and results in degradation of the skin barrier function.

Usually, skin adhesive keeps the skin dry by being water permeable. This allows moisture to transport through the adhesive from the skin side to the outer side, where it is allowed to evaporate. This mechanism is not usable for ostomy skin adhesives, because a water impermeable layer covers the outer side of an ostomy adhesive. The water impermeable layer prevents ostomy discharge to enter the adhesive from the outside. Thus, evaporation of moisture is not possible. Hence, adhesive compositions used for ostomy appliances are made water absorbent. Absorbing particles or hydrocolloids (HC) are mixed into a hydrophobic adhesive matrix to absorb moisture from the skin and thereby keeping the skin relatively dry. This technique is well known in the art (see for example US Patent No. 6,451,883) and forms the basis for all commercially available ostomy adhesives.

The adhesive matrix, in traditional state of the art of ostomy adhesives without HC particles, is very hydrophobic with very low water permeability. The only way water can transmit within the adhesive is through the hydrocolloid particles that are mixed therein. As these particles are much smaller than the total thickness of the adhesive layer, the only way water can migrate into the adhesive is if the HC particles touch each other and form bridges for water permeation. This limit in water transportation dictates a relatively high loading of hydrocolloid particles in the adhesive, in a way that enough particles touch each other. As the particles used are hard relative to the adhesive matrix, this addition of a large quantity of particles makes the adhesive hard and uncomfortable for the user.

By using a water permeable adhesive matrix such as the adhesive described in International Publication No. WO 05/032401, the water transportation is less dependent on the number of particles that touches each other, fewer particles are needed to ensure proper water mobility in the adhesive.

Unfortunately, by reducing the amount of absorbing particles, the absorption capacity and rate is reduced. Water absorption in the adhesive is driven by a difference in vapour pressure between the skin and the inside of the adhesive. The vapour pressure over hydrocolloids grows rapidly towards the equilibrium vapour pressure of water, in that water is absorbed by the particles. As vapour pressure grows in the hydrocolloids, the driving force reduces and the water transport gets slower. This would also be the case for a regular HC adhesive with impermeable adhesive matrix, but here, water transport is helped by the expanding particles starting to touch more neighbour particles and form more bridges. In this way the resistance against water flux reduces and compensates for the lower driving force.

Thus, reducing the amount of particles not only reduces the absorption capacity, but also reduces the absorption rate of the adhesive. Using the known technology today, it is not possible to make an ostomy adhesive that is soft, has high water absorption capacity and also has a relatively high and constant transient water uptake. WO 2007/128320 relates to a pressure sensitive adhesive composition consisting of a continuous phase comprising a cross-linked polyalkyleneoxide polymer, such as polypropyleneoxide, and a discontinuous phase comprising water absorbent hydrophilic agents, selected from water soluble hydrocolloids. The document does not disclose a continuous phase with a polymer selected from polyurethane, silicone, and ethylene vinyl acetate; it also does not disclose water soluble compounds selected from polypeptides, inorganic acids, amino acids, amines, and urea, all with a molecular weight less than 200 g/mol. The present invention provides an absorbing adhesive with very low particle loading and still having high water absorption capacity.

### Summary of the Invention

Accordingly, the present invention relates to a pressure sensitive adhesive composition comprising a continuous phase and a discontinuous phase wherein

a) the continuous phase comprises a water vapour permeable hydrophobic polymer; selected from the group of polyurethane, silicone, ethylene vinyl acetate, and mixtures thereof and

b) the discontinuous phase comprises water soluble compound particles selected from the group of polypeptides, inorganic acids, amino acids, amines, urea, and mixtures thereof with a molecular weight below 200 g/mol, wherein the water vapour permeable hydrophobic polymer absorbs less than 5 wt-% at equilibrium and has a moisture vapour transmission rate greater than 20 g/m²/24.

**Detailed description of the Invention**

The present invention relates to a pressure sensitive adhesive composition comprising a continuous phase and a discontinuous phase wherein

a) the continuous phase comprises a water vapour permeable hydrophobic polymer; and

b) the discontinuous phase comprises a water soluble compound.

As used herein, the total adhesive composition means the discontinuous phase and the continuous phase in combination.
As used herein the discontinuous phase means the water soluble compound(s) or mixture of such compounds and any other solid materials, preferably in particulate form, such as filler (native starch), colours, hydrocolloids etc. which are distributed in the continuous phase.
As used herein the continuous phase means the total adhesive composition except the discontinuous phase.
The present invention provides an absorbing adhesive with very low particle loading and still high water absorption capacity. These very attractive features are obtained by using low molecular weight water-soluble compounds instead of water absorbent particles as absorbers. Furthermore, it is required that the adhesive matrix is water vapour permeable and substantially impermeable. In this way, water vapour can diffuse towards the compound particle and slowly dissolve it, but the material from the dissolved compound cannot escape the adhesive matrix.
Usually low molecular weight water-soluble compounds cannot be used as absorbing particles because upon water contact, they dissolve and will leach out of the polymer matrix.
Surprisingly, the water soluble compound and permeable polymer combination can absorb moisture without the negative effect of the dissolved material leaching out. One theory of how this works is that the polymer and compound distribution provide an osmotic effect and moisture is transported through the vapour permeable, but liquid impermeable polymer acts as a membrane. The liquid impermeable polymer also has a function of sealing the dissolved material until the pressure balance is changed sufficiently. This, in normal use of these adhesives, is never reached, therefore the transport is essentially in one direction only. As long as there is no tunnel or opening leading directly to the surfaces of the adhesive, the dissolved material cannot leach out.

A compound particle in an adhesive matrix "absorbs" in a fundamentally different way than a regular hydrocolloid in an adhesive matrix:

First, the amount of water that a low molecular weight water-soluble compound can absorb, at a given partial pressure of water, can be huge compared to the hydrocolloid. For example, at room temperature at 75% relative humidity (RH), a low molecular weight water-soluble compound made of Urea can absorb more than its own weight, whereas a hydrocolloid like Aquasorb A800 only absorbs about 0,2 times its own weight. Thus, Urea has a water absorption capacity that is an order of magnitude higher than hydrocolloids.

Next, the vapour pressure over a low molecular weight water-soluble compound particle is independent of the amount of absorbed water until the material is completely dissolved. This is contradictory to the hydrocolloid particle where equilibrium vapour pressure increases rapidly with the amount of absorbed water. When the vapour pressure increases, the difference between vapour pressure on the skin and the adhesive is reduced and thus the driving force of water transport is reduced. This difference in the vapour pressure effects makes the absorption rate of an adhesive with low molecular weight water-soluble compounds more constant in time and less a function of the amount of absorbed water.
Finally, the mechanical behaviour of the adhesive also changes during absorption of water. When no water is absorbed in an adhesive with low molecular weight water-soluble compounds in it, the adhesive will be harder than the equivalent adhesive without particles. This is because the particles are harder than the adhesive matrix. However, as the adhesive starts to absorb water, the particles are dissolved and the adhesive gets softer and softer.
A surprising benefit of the compound particle turning to liquid form upon dissolution but still being kept in pockets within the polymer matrix, is that the adhesive moduli are reduced. The adhesive becomes softer and even more comfortable to wear. But there is no reduction in erosion resistance.
From the physics of dissolving materials, it is possible, to a first order approximation, to calculate the minimum solubility of the material to achieve satisfactory absorption rates. Raoult's law teaches that the equilibrium vapour pressure of a species (in the present case: water) over a solution containing the species is equal to the saturated vapour pressure of the pure species multiplied by its mole fraction in the solution.

$$p_{H_2O} = x_{H_2O}\, p_{H_2O}^{sat}$$

According to the invention, it is desired that the vapour pressure in the particles is less than 98% of pure water (very close to isotonic water), because a vapour pressure gradient between skin and adhesive is needed to have water transportation into the adhesive. From the minimum vapour pressure requirement according to the invention and Raoult's law it is possible to calculate the minimum compound solubility:

$$0.98 > \frac{p_{H_2O}}{p_{H_2O}^{sat}} = x_{H_2O} = \frac{n_{H_2O}}{n_{H_2O} + n_{compound}}$$

Where $p_{H_2O}$ is partial pressure of water, $p_{H_2O}^{sat}$ is vapour pressure of pure water, $x_{H_2O}$ is the molar fraction of water and $n_{H_2O}$ and $n_{compound}$ are molar concentrations of water and compound.

According to this equation, the minimum solubility of the compound is calculated to be 1.13mol/L $H_2O$. The water soluble compounds used according to the invention are selected from the group of polypeptides, inorganic acids, amino acids, amines, urea, and mixtures thereof.

The term "urea" used herein should be understood to comprise urea as well as which has been N-substituted or N,N-disubstituted by lower alkyl.

"Lower alkyl" is used in the present context to designate straight or branched or cyclic aliphatic groups having from 1 to 6 carbon atoms, preferably from 1 to 4 carbon atoms such as methyl, ethyl, propyl, isopropyl or n, iso or tert.butyl. The

molecular weight of the water soluble compound is below 200 g/mol.

The compound or mixture of compounds is preferably non-toxic and insoluble in the adhesive matrix. The compound is preferably a solid.

According to one embodiment of the invention, the water soluble compound is fully soluble at 1 mole per litre.

According to one embodiment of the invention, the content of the water soluble compound is below 40 wt% of the total pressure sensitive adhesive composition. The pressure sensitive adhesive composition comprises water vapour permeable hydrophobic polymer from the group of polyurethane, silicone, ethylene vinyl acetate, and mixtures thereof.

As used herein a water vapour permeable hydrophobic polymer means a polymer that absorbs less than 5% in wt, preferably less than 1%, at equilibrium and has a moisture vapour transmission rate of greater than 20 g/m$^2$/24hrs, preferably greater than 100 g/m$^2$/24hrs.

In one embodiment of the invention, the water vapour permeable hydrophobic polymer is crosslinked. As used herein a crosslink means a small region in a macromolecule (polymer chain structure) from which more than 2 chains emanate. The linking may be covalent, physical or ionic. The water vapour permeable hydrophobic polymer has a permeability above 20 g/m$^2$/24h.

In another embodiment of the invention, the water vapour permeable hydrophobic polymer is a block copolymer.

As used herein a block copolymer means a copolymer in which the repeating units in the main chain occur in blocks, e.g.,-(a)m-(b)n-(a)p-(b)q- ,where a and b represent the repeating units.

In a preferred embodiment of the invention, the water vapour permeable hydrophobic polymer is polyurethane.

In a preferred embodiment of the invention, the water vapour permeable hydrophobic polymer is silicone.

In a preferred embodiment of the invention, the water vapour permeable hydrophobic polymer is ethylene vinyl acetate.

Preferred particle size of the discontinuous phase is as small as possible, smaller particles are more difficult to see by the naked eye and will give products that are more pleasing to the eye. An upper limit on particle size is the size of the smallest dimension of the adhesive. Thus, a 300$\mu$m thick adhesive should not contain particles with diameters above 300$\mu$m. There is a tendency of the hygroscopic particles to agglomerate and this effect will increase with decreasing particle size. Therefore, a preferred particle size would be from 10-300$\mu$m. Also, the particles may contain an anti agglomerating agent to reduce agglomeration of small particles.

The pressure sensitive adhesive composition according to the invention may contain other conventional ingredients for adhesive compositions, such as tackifiers, extenders, non-reactive polymers, oils (e.g. polypropylenoxide, ethyleneoxide-propyleneoxide copolymers, mineral oil), plasticisers, fillers, surfactants. The adhesive may also comprise pharmaceutically active ingredients. These optional ingredients may be present in the reaction mixture during the cross linking reaction.

In one embodiment of the invention, the pressure sensitive adhesive composition further comprises hydrocolloid.

In a preferred embodiment of the invention, the amount of hydrocolloid is below 50% w/w of the total composition. In another embodiment of the invention, a layered adhesive construct comprising a backing layer and at least one layer of a pressure sensitive adhesive composition according to the invention.

The adhesive according to the invention may be foamed into foamed adhesive in a number of ways, either chemically or mechanically.

Chemical blowing agents or other materials added to the adhesive formula itself may generate gas bubbles by a variety of mechanisms. These mechanisms include but are not limited to chemical reaction, physical changes, thermal decomposition or chemical degradation, leaching of a dispersed phase, volatilisation of low boiling materials or by a combination of these methods.

Any of the commercially known chemical blowing agents may be used. The chemical blowing agents are suitably non-toxic, skin friendly and environmentally safe, both before and after decomposition.

The amount of chemical blowing agent to be added to the adhesive mixture may range from about 0,01% up to about 90% by weight, with a practical range including about 1% up to about 20% by weight. The amount of gas to be added may be determined by measuring the amount of gas generated from a candidate mixture and calculating the amount of foaming required for the final product, tempered by experience of the amount of gas lost to atmosphere during the foaming process.

Another method for creating a foamed adhesive of the invention is a method where a mechanical process is used to add a physical blowing agent, similar to whipping the adhesive mass into froth, thus creating a foamed structure. Many processes are possible including processes involving incorporation of air, nitrogen, carbon dioxide, or other gases or low boiling point volatile liquids during the manufacturing process for the adhesive.

The invention also relates to medical devices comprising a pressure sensitive adhesive composition as described above.

The medical device comprising an adhesive composition according to the invention may be an ostomy appliance, a dressing (including wound dressings), a wound drainage bandage, a skin protective bandage, a device for collecting urine, an orthose or a prosthese, e.g. a breast prothesis, and electronic device such as a measuring instrument or a power source, such as a battery.

The medical device may also be a tape (e.g. an elastic tape or film), or a dressing or a bandage, for securing a medical device, or a part of the medical device to the skin, or for sealing around a medical device attached to the skin.

The medical device may in its simplest construction be an adhesive construction comprising a layer of the pressure sensitive adhesive composition according to the invention and a backing layer.

The backing layer is suitably elastic (has a low modulus), enables the adhesive construction to conform to the skin movement and provide comfort when using it.

The thickness of the backing layer used according to the invention is dependent on the type of backing used. For polymer films, such as polyurethane films, the overall thickness may be between 10 to 100 $\mu$m, preferably between 10 to 50 $\mu$m, most preferred about 30 $\mu$m.

In one embodiment of the invention, the backing layer is non-vapour permeable.

In another embodiment of the invention, the backing layer is water vapour permeable and has a moisture vapour transmission rate above 500g/m$^2$/24h. In this case the adhesive construction of the invention may provide a good moisture absorption rate and absorption capacity and is able to transport a large quantity of moisture through the construction and away from the skin. Both the chemical composition and physical construction of the adhesive layer and the chemical and physical construction of the backing layer affect the water vapour permeability. With regard to the physical construction, the backing layer may be continuous (no holes, perforations, indentations, no added particles or fibers affecting the water vapour permeability) or discontinuous (it has holes, perforations, indentations, added particles or fibers affecting the water vapour permeability).

The moisture vapour transmission rate of the backing layer is suitably above 500 g/m$^2$/24h, most preferably above 1,000 g/m$^2$/24h, even more preferred above 3,000 and most preferred above 10,000.

The adhesive composition according to the invention is suitable for fastening a prosthetic device to the skin. For example breast prosthesis could be fastened using adhesives described in this patent. The adhesive composition according to the invention is superior to state of the art because it can keep the skin dry by absorbing moisture.

The adhesive composition is completely elastic in its nature. It only absorbs water and no absorbent is allowed to leak out. Thus, deformations are reversible through the elasticity of the adhesive and water absorption is reversible by drying. The adhesive composition according to the invention is therefore very suitable as a reusable adhesive.

An ostomy appliance of the invention may be in the form of an adhesive wafer forming part of a two-piece appliance or in the form of a one-piece appliance comprising a collecting bag for collecting the material emerging from the stoma and an adhesive flange for attaching the one-piece appliance to the skin of the ostomate. A separate collecting bag may be attached to the adhesive wafer by any manner known per se, e.g. through mechanical coupling using a coupling ring or through use of adhesive flanges. The adhesive according to this invention may be one of more layers that make up the total adhesive sheet.

In another embodiment of the invention, the adhesive is part of a faecal-collecting device, attaching a bag or another collecting device to the perianal skin.

A dressing or adhesive sheet of the invention may have bevelled edges in order to reduce the risk of "rolling-up" the edge of the dressing reducing the wear-time. A bevelling may be carried out discontinuously or continuously in a manner known per se e.g. as disclosed in EP Patent No. 0 264 299 or US Patent No. 5,133,821.

## Methods

Determination of moisture vapour transmission rate (MVTR)

MVTR is measured in grams per square meter (g/m$^2$) over a 24 hours period using an inverted cup method.

A container or cup being water and water vapour impermeable having an opening is used. 20ml saline water (0.9%NaCl in demineralised water) is placed in the container and the opening is sealed with the test adhesive film. The container, with a duplicate, is placed into an electrically heated humidity cabinet and the container or cup is placed upside down, in a way that the water is in contact with the adhesive. The cabinet is maintained at 37°C and 15% relative humidity (RH). After about an hour, the containers are considered to be in equilibrium with the surroundings and it is weighed. 24h after the first weighing, the containers are weighed again. The difference in weight is due to evaporation of vapour transmitted through the adhesive film. This difference is used to calculate Moisture vapour transmission rate or MVTR. MVTR is calculated as the weight loss after 24h divided by the area of the opening in the cup (g/m$^2$/24h). The MVTR of a material is a linear function of the thickness of the material. Thus, when reporting MVTR to characterise a material, it is important to state the thickness of the material. We use 150$\mu$m as a reference. If thinner or thicker samples have been measured, the MVTR is reported as corresponding to a 150$\mu$m sample. Thus a 300$\mu$m sample with a measured MVTR of 10g/m$^2$/24h is reported as having MVTR=20g/m$^2$/24h for a 150$\mu$m sample because of the linear connection between thickness of sample and MVTR of sample. Finally, we note that by using this method, we introduce an error by using a supporting PU film. However, the water permeability of the used film is very high (10,000g/m$^2$/24h) and the error that is introduced is very small.

Determination of water absorption

Pieces of adhesive of 1x25x25 mm$^3$ were immersed in saline water (0,9% NaCl in demineralised water) at 37°C. The samples were removed and carefully dripped dry and weighed after 30, 60, 90, 120, 240, and 1,440 hours. The change in weight is recorded and reported as weight gain in g/cm$^2$.

**Claims**

1. A pressure sensitive adhesive composition comprising a continuous phase and a discontinuous phase wherein

   a) the continuous phase comprises a water vapour permeable hydrophobic polymer selected from the group of polyurethane, silicone, ethylene vinyl acetate, and mixtures thereof; and
   b) the discontinuous phase comprises water soluble compound particles selected from the group of polypeptides, inorganic acids, amino acids, amines, urea, and mixtures thereof with a molecular weight below 200 g/mol,

   wherein the water vapour permeable hydrophobic polymer absorbs less than 5 wt-% at equilibrium and has a moisture vapour transmission rate greater than 20 g/m$^2$/24h.

2. The pressure sensitive adhesive composition according to claim 1, wherein the water vapour permeable hydrophobic polymer is crosslinked.

3. The pressure sensitive adhesive composition according to any of claims 1-2, wherein the water vapour permeable hydrophobic polymer is polyurethane.

4. The pressure sensitive adhesive composition according to any of claims 1-2, wherein the water vapour permeable hydrophobic polymer is silicone.

5. The pressure sensitive adhesive composition according to any of claims 1-2, wherein the water vapour permeable hydrophobic polymer is ethylene vinyl acetate.

6. The pressure sensitive adhesive composition according to any of claims 1-5, wherein the water soluble compound particles are selected from the group of amino acids, glycine, urea, and mixtures thereof.

7. The pressure sensitive adhesive composition according to any of claims 1-6, wherein the water soluble compound particles are fully soluble at 1 mole per litre.

8. The pressure sensitive adhesive composition according to any of claims 1-7, wherein the content of the water soluble compound particles is below 40 wt% of the total pressure sensitive adhesive composition.

9. A layered adhesive construct comprising a backing layer and at least one layer of a pressure sensitive adhesive composition according to any of claims 1-8.

10. A medical device comprising a pressure sensitive adhesive composition according to any of claims 1-8 and a backing layer.

11. The medical device according to claim 10, wherein the backing layer is non-vapour permeable.

12. The medical device according to claim 10, wherein the backing layer is water vapour permeable and has a moisture vapour transmission rate above 500 g/m$^2$/24h.

13. The medical device according to any of claims 10-12, wherein the medical device is a dressing, an ostomy appliance, a medical tape, or a dressing or bandage for sealing around a medical device on the skin.

## Patentansprüche

1. Haftklebstoff zusammensetzung, umfassend eine kontinuierliche Phase und eine diskontinuierliche Phase, wobei

   a) die kontinuierliche Phase ein wasserdampfdurchlässiges hydrophobes Polymer aus der Gruppe Polyurethane, Silikon, Ethylen-Vinylacetat und Mischungen davon umfasst und
   b) die diskontinuierliche Phase Teilchen einer wasserlöslichen Verbindung aus der Gruppe Polypeptide, anorganische Säuren, Aminosäuren, Aminen, Harnstoff und Mischungen davon mit einem Molekulargewicht von weniger als 200 g/mol umfasst,

   wobei das wasserdampfdurchlässige hydrophobe Polymer im Gleichgewicht weniger als 5 Gew.-% absorbiert und eine Wasserdampfdurchlässigkeit von mehr als 20 $g/m^2/24$ h aufweist.

2. Haftklebstoffzusammensetzung nach Anspruch 1, wobei das wasserdampfdurchlässige hydrophobe Polymer vernetzt ist.

3. Haftklebstoffzusammensetzung nach einem der Ansprüche 1-2, wobei es sich bei dem wasserdampfdurchlässigen hydrophoben Polymer um Polyurethan handelt.

4. Haftklebstoffzusammensetzung nach einem der Ansprüche 1-2, wobei es sich bei dem wasserdampfdurchlässigen hydrophoben Polymer um Silikon handelt.

5. Haftklebstoffzusammensetzung nach einem der Ansprüche 1-2, wobei es sich bei dem wasserdampfdurchlässigen hydrophoben Polymer um Ethylen-Vinylacetat handelt.

6. Haftklebstoffzusammensetzung nach einem der Ansprüche 1-5, wobei die Teilchen der wasserlöslichen Verbindung aus der Gruppe Aminosäuren, Glycin, Harnstoff und Mischungen davon ausgewählt sind.

7. Haftklebstoffzusammensetzung nach einem der Ansprüche 1-6, wobei die Teilchen der wasserlöslichen Verbindung bei 1 mol pro Liter vollständig löslich sind.

8. Haftklebstoffzusammensetzung nach einem der Ansprüche 1-7, wobei der Gehalt der Teilchen der wasserlöslichen Verbindung weniger als 40 Gew.-%, bezogen auf die gesamte Haftklebstoffzusammensetzung, beträgt.

9. Schichtförmiges Klebstoffkonstrukt, umfassend eine Trägerschicht und mindestens eine Schicht einer Haftklebstoffzusammensetzung nach einem der Ansprüche 1-8.

10. Medizinische Vorrichtung, umfassend eine Haftklebstoffzusammensetzung nach einem der Ansprüche 1-8 und eine Trägerschicht.

11. Medizinische Vorrichtung nach Anspruch 10, wobei die Trägerschicht nicht dampfdurchlässig ist.

12. Medizinische Vorrichtung nach Anspruch 10, wobei die Trägerschicht wasserdampfdurchlässig ist und eine Wasserdampfdurchlässigkeit von mehr als 500 $g/m^2/24$ h aufweist.

13. Medizinische Vorrichtung nach einem der Ansprüche 10-12, wobei es sich bei der medizinischen Vorrichtung um einen Verband, eine Ostomievorrichtung, ein medizinisches Klebeband oder einen Verband oder eine Bandage zur Versiegelung um eine medizinische Vorrichtung herum auf der Haut handelt.

## Revendications

1. Composition adhésive autocollante comprenant une phase continue et une phase discontinue dans laquelle

   a) la phase continue comprend un polymère hydrophobe perméable à la vapeur d'eau choisi dans le groupe du polyuréthane, de la silicone, de l'éthylène-acétate de vinyle et des mélanges de ceux-ci ; et
   b) la phase discontinue comprend des particules de composé hydrosoluble choisies dans le groupe des polypeptides, des acides inorganiques, des acides aminés, des aminés, de l'urée et des mélanges de ceux-ci ayant une masse moléculaire au-dessous de 200 g/mol,

   dans laquelle le polymère hydrophobe perméable à la vapeur d'eau absorbe moins de 5 % en poids à l'équilibre et a un taux de transmission de la vapeur d'eau supérieur à 20 $g/m^2/24$ h.

2. Composition adhésive autocollante selon la revendication 1, dans laquelle le polymère hydrophobe perméable à la vapeur d'eau est réticulé.

3. Composition adhésive autocollante selon l'une quelconque des revendications 1-2, dans laquelle le polymère hydrophobe perméable à la vapeur d'eau est du polyuréthane.

4. Composition adhésive autocollante selon l'une quelconque des revendications 1-2, dans laquelle le polymère hydrophobe perméable à la vapeur d'eau est de la silicone.

5. Composition adhésive autocollante selon l'une quelconque des revendications 1-2, dans laquelle le polymère hydrophobe perméable à la vapeur d'eau est de l'éthylène-acétate de vinyle.

6. Composition adhésive autocollante selon l'une quelconque des revendications 1-5, dans laquelle les particules de composé hydrosoluble sont choisies dans le groupe des acides aminés, de la glycine, de l'urée et des mélanges de ceux-ci.

7. Composition adhésive autocollante selon l'une quelconque des revendications 1-6, dans laquelle les particules de composé hydrosoluble sont totalement solubles à 1 mole par litre.

8. Composition adhésive autocollante selon l'une quelconque des revendications 1-7, dans laquelle la teneur des particules de composé hydrosoluble est au dessous de 40 % en poids de la composition adhésive autocollante totale.

9. Construction adhésive en couches comprenant une couche de support et au moins une couche de composition adhésive autocollante selon l'une quelconque des revendications 1-8.

10. Dispositif médical comprenant une composition adhésive autocollante selon l'une quelconque des revendications 1-8 et une couche de support.

11. Dispositif médical selon la revendication 10, dans lequel la couche de support est non perméable à la vapeur.

12. Dispositif médical selon la revendication 10, dans lequel la couche de support est perméable à la vapeur d'eau et a un taux de transmission de la vapeur d'eau au-dessus de 500 g/m$^2$/24 h.

13. Dispositif médical selon l'une quelconque des revendications 10-12, le dispositif médical étant un pansement, un appareillage pour stomie, un ruban adhésif médical ou un pansement ou bandage pour assurer l'étanchéité autour d'un dispositif médical sur la peau.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6451883 B
- WO 05032401 A
- WO 2007128320 A
- EP 0264299 A
- US 5133821 A


**Non-patent literature cited in the description**

- **MAIN, K. ; K. O. NILSSON ; N. E. SKAKKEBAEK.** Influence of sex and growth hormone deficiency on sweating, Scand. *J. Clin. Lab Invest,* 1991, vol. 51, 475-480